# EUROPÄISCHE PATENTSCHRIFT

(11) **EP 3 122 452 B1**
(45) Veröffentlichungstag und Bekanntmachung des Hinweises auf die Patenterteilung: **27.10.2021**
(21) Anmeldenummer: 15710525.5
(22) Anmeldetag: 19.03.2015
(51) Int. Cl.: B01J 23/80, B01J 35/00, B01J 35/02, B01J 35/10, B01J 37/00, B01J 37/08, B01J 37/06, B01J 37/03, C07C 31/04

(54) **VERFAHREN ZUR HERSTELLUNG VON KATALYSATOREN MIT ERHÖHTER FESTIGKEIT UND VERRINGERTEM VOLUMENSCHWUND**
METHOD FOR PRODUCING CATALYSTS HAVING INCREASED STRENGTH AND DECREASED VOLUME REDUCTION
PROCÉDÉ DE PRODUCTION DE CATALYSEURS AYANT UNE RÉSISTANCE ACCRUE ET UN FAIBLE RÉTRÉCISSEMENT VOLUMIQUE

(30) Priorität: 26.03.2014 DE 102014004391
(43) Veröffentlichungstag der Anmeldung: 01.02.2017
(73) Patentinhaber: Clariant International Ltd, 4132 Muttenz (CH)
(72) Erfinder: KURR, Patrick, 41061 Mönchengladbach (DE); KNIEP, Benjamin, 83064 Raubling (DE); PAA, Andrea, 83059 Kolbermoor (DE); PRITSCHER, Verena, 83209 Prien (DE)
(74) Vertreter: Kuba, Stefan
(86) Internationale Anmeldenummer: PCT/EP2015/055759
(87) Internationale Veröffentlichungsnummer: WO 2015/144549

(56) Entgegenhaltungen:
- EP-A1- 2 357 037
- EP-A2- 2 305 379
- WO-A1-2014/048957

## Beschreibung

Die Erfindung betrifft ein Verfahren zur Herstellung von kupferhaltigen Katalysatoren, insbesondere von Katalysatorformkörper mit erhöhter mechanischer Festigkeit und geringer Volumenschrumpfung, sowie die durch das erfindungsgemäße Verfahren hergestellten Katalysatorformkörper und deren Verwendung als Katalysatoren bzw. als Präkursoren und Komponenten für Katalysatoren. Die erfindungsgemäßen Katalysatoren eignen sich insbesondere für die Methanolsynthese und für die Tieftemperatur-Konvertierung von CO zu CO₂.

### Hintergrund der Erfindung

Kupferhaltige Katalysatoren werden in großem Maßstab bei der Herstellung von Grund- und Feinchemikalien eingesetzt, z.B. in der katalytischen Umsetzung von Gemischen aus CO₂, CO und H₂ zu Methanol. Die Eigenschaften solcher Katalysatoren können in Abhängigkeit von unterschiedlichen Parametern variiert werden, wie z.B. durch die Wahl des Trägermaterials oder durch die Größe und Form der Metallteilchen. Die Aktivität dieser Katalysatoren mit Kupfer als aktiver Komponente ist dabei in der Regel abhängig von der Größe der Metallpartikel.

Kupferhaltige Katalysatoren werden oft mittels eines mehrstufigen Verfahrens hergestellt. Dabei wird in einem ersten Schritt ein Katalysatorvorläufermaterial aus der Kupferkomponente sowie weiteren Komponenten, die eine stabilisierende Trägerfunktion im späteren Katalysator auf die Aktivkomponente haben, hergestellt. Üblicherweise erfolgt dies durch Co-Fällung aller gewünschten Komponenten. Nach dem Waschen zum Entfernen von überschüssigen Salzen bzw. unerwünschten (Alkali-) Metallen erfolgt eine Trocknung zu einem festen Katalysatorvorläufermaterial. In einem weiteren Schritt wird dieser feste Katalysatorvorläufer thermisch behandelt und in einen größtenteils oxidischen Zustand überführt. Anschließend erfolgt die Formgebung der Katalysatormasse durch Tablettieren, Granulieren, Extrudieren oder durch eine Kombination aus den genannten Methoden. Schließlich wird der erhaltende Formkörper mittels Wasserstoff, Kohlenmonoxid oder nasschemischer Reduktionsmittel in das katalytisch aktive, hochdisperse Kupfermetall überführt.

Üblicherweise werden Methanolsyntheseanlagen mit dem oxidischen Katalysator in Tablettenform beladen und anschließend vor Ort mittels Reduktion in einem Wasserstoffstrom nach vorgebenden Aktivierungsverfahren in den katalytisch aktiven Katalysator überführt.

Beispielsweise beschreiben die DE 10 2005 020 630-A1, die WO 03/053569, die DE 3317 725 A1 sowie die DE 101 60 487 A1 die Herstellung von kupferbasierten Katalysatoren für die Synthese von Methanol.

Die entsprechend hergestellten Katalysatoren haben jedoch den Nachteil, dass sie eine ausgeprägte Volumenschrumpfung durch die Reduktion, die darüber hinaus mit einem deutlichen Verlust der mechanischen Festigkeit des Formkörpers verbunden ist, erleiden. Die Gründe für die ausgeprägte Volumenschrumpfung von kupferhaltigen Katalysatorformkörpern während der Reduktion sind vielfältig. Beispielsweise kann der Formkörper bedingt durch die Volumenkontraktion beim Übergang vom oxidischen in den metallischen Zustand schrumpfen. Weitere Gründe liegen in der Kondensation von Wasserdampf, der bei der Reduktion üblicherweise entsteht, was zum Kollabieren des Porengefüges des Katalysatorformkörpers führen kann. Für eine optimale Ausnutzung der Katalysatorschüttung im Reaktor im Fahrbetrieb ist aber eine möglichst geringe Volumenschrumpfung des Formkörpers gewünscht. Eine verstärkte Volumenschrumpfung der Katalysatorschüttung im Reaktor führt zu einer schlechteren Ausnutzung des Reaktors - ein Teil des Reaktors bliebe leer - sowie zu einer schlechteren Ausnutzung der Wärmeaustauschfläche des Reaktors. Letzteres ist besonders problematisch, da die Kühlung der Katalysatorschüttung üblicherweise einen limitierenden Faktor im Fahrbetrieb darstellt.

Prinzipiell kann der Reduktionsschritt auch vor dem Beladen des Reaktors mit dem Katalysator durch Reduktion und anschließender, schonender Passivierung mit einem Oxidationsmittel, im Allgemeinen mit gasförmigem Sauerstoff (Reduktionsstabilisierung) oder durch nasschemische Stabilisierung mittels eines Öls erfolgen. Die so hergestellten Kupferkatalysatoren haben jedoch üblicherweise die Nachteile, dass (i) ein weiterer mit zusätzlichen Kosten verbundener Prozessschritt bei der Katalysatorherstellung notwendig ist, dass (ii) der Kupferkatalysator eine gegenüber dem unreduzierten (oxidischen) Zustand deutlich geringere mechanische Festigkeit aufweist, was sich insbesondere bei der Reaktorbeladung durch verstärken Bruch im Vergleich zum oxidischen Zustand in der Katalysatorschüttung bemerkbar macht, und dass (iii) die reduktionsstabilisierten Katalysatoren nicht lagerstabil sind und mit der Zeit durch Kontakt mit der Luft reoxidieren. Hier sei beispielsweise auf die Schrift EP 1 238 702 A1 verwiesen. EP 2 357 037 A1 offenbart ein Katalysator mit verbesserten Seitendruckfestigkeit, enthaltend Cu, Zn und Al.

Von Formkörpern, wie z.B. Tabletten, wird eine große mechanische Festigkeit verlangt, damit sie die Belastungen, die auf sie zum Zeitpunkt der Reaktorbefüllung sowie im Fahrbetrieb einwirken, unbeschadet überstehen können. Im Allgemeinen ist aber mit der Reduktion von Katalysatoren auch eine deutliche Verringerung der mechanischen Festigkeit verbunden. Insbesondere bei reduzierten Metallkatalysatoren in Form von Tabletten ist die Seitendruckfestigkeit - als Maß für die mechanische Festigkeit von Tabletten - im Unterschied zur Seitendruckfestigkeit im oxidischen Zustand um ein Vielfaches geringer. Entsprechend ist auch die mechanische Stabilität der Tabletten nach der Reduktion um ein Vielfaches geringer als im oxidischen Zustand. Durch Vibrationen, äußere und innere im Reaktor vorliegende Druckschwankungen im Fahrbetrieb und/oder durch die Last der Katalysatorschüttung auf den einzelnen Katalysatorformkörper werden die Tabletten im Fahrbetrieb durch Reibung mit der Reaktorwand oder durch Reibung der Tabletten untereinander stark beansprucht, was insbesondere durch die an den Kanten und Ecken der Tabletten verursachten Spannungen zu einem verstärktem Abrieb an den Tabletten führt.

Die Aufgabe der vorliegenden Erfindung besteht somit darin, ein Herstellungsverfahren bereitzustellen, mit dem kupferhaltige Katalysatorformkörper mit stark verringerter, vorzugsweise gar keiner Volumenschrumpfung nach der Aktivierung (Reduktion zum Metall) bei gleichzeitig hoher mechanischer Festigkeit erhalten werden können. Das Verfahren soll außerdem leicht durchführbar und kostengünstig sein. Die erhaltenen Katalysatoren sollen bevorzugt zur Synthese von Methanol einsetzbar sein.

### Zusammenfassung der Erfindung

Die Erfindung betrifft ein Verfahren zur Herstellung eines Katalysatorformkörpers, enthaltend Kupfer, Zink und Aluminium, umfassend die folgenden Schritte:
(a) Vereinigen einer alkalischen Lösung, insbesondere eines carbonathaltigen Fällungsmittels, mit einer kupferhaltigen Lösung, die erhältlich ist durch Lösen und/oder Suspendieren von einer Kupferverbindung, einer Zinkverbindung und einer Aluminiumverbindung, zum Erhalt eines Niederschlags;
(b) Abtrennen, gegebenenfalls Waschen und/oder gegebenenfalls Trocknen des Niederschlags zum Erhalt eines festen Katalysatorvorläufers;
(c) thermische Behandlung von in Schritt (b) erhaltenem festen Katalysatorvorläufer bei einer Temperatur im Bereich von 200°C bis 600°C, zum Erhalt eines Mischoxids, mit einer BET-Oberfläche im Bereich von 80 m²/g bis 140 m²/g, bevorzugt im Bereich von 85 m²/g bis 120 m²/g, besonders bevorzugt im Bereich von 90 m²/g bis 110 m²/g;
(d) Mischen von in Schritt (b) erhaltenem festen Katalysatorvorläufer mit in Schritt (c) erhaltenem Mischoxid in einem Gewichtsverhältnis von festem Katalysatorvorläufer zu Mischoxid im Bereich von 2 : 98 bis 20 : 80, vorzugsweise im Bereich von 5 : 95 bis 15 : 85, bevorzugter im Bereich von 10 : 90 bis 15 : 85 zum Erhalt einer Mischung; und
(e) Tablettieren der in Schritt (d) erhaltenen Mischung, wobei der Katalysatorformkörper eine auf das Tablettengewicht bezogene Seitendruckfestigkeit von 500 N/g oder mehr, bevorzugt von 600 N/g oder mehr, insbesondere im Bereich von 600 bis 900 N/g aufweist.

Darüber hinaus betrifft die Erfindung Katalysatorformkörper, die sich durch die erfindungsgemäßen Verfahren herstellen lassen. Außerdem betrifft die Erfindung die Verwendung von durch das erfindungsgemäße Verfahren hergestellten Katalysatorformkörper für die Synthese von Methanol aus Synthesegas enthaltend CO₂, CO, und H₂ oder für die Tieftemperatur-Konvertierung von CO zu CO₂.

### Detaillierte Beschreibung der Erfindung

Das erfindungsgemäße Verfahren zur Herstellung eines Katalysatorformkörpers umfasst in einem Schritt (a) das Vereinigen einer kupferhaltigen Lösung mit einer alkalischen Lösung unter Bildung eines Niederschlags.

Die kupferhaltige Lösung wird hergestellt, indem eine Kupferverbindung, eine Zinkverbindung und eine Aluminiumverbindung in einem Behälter in einem geeigneten Lösungsmittel gelöst und/oder suspendiert werden. Alternativ können eine Kupferverbindung, eine Zinkverbindung und eine Aluminiumverbindung in mehreren Behältern gelöst und/oder suspendiert werden und die erhaltenen Lösungen zu der kupferhaltigen Lösung vereinigt werden.

Die Formulierung "Lösung" im Sinne der vorliegenden Erfindung schließt sowohl Lösungen als auch Suspensionen und Aufschlämmungen ein, wobei Lösungen und Suspensionen bevorzugt sind.

Vorzugsweise ist das Lösungsmittel für die Herstellung der kupferhaltigen Lösung (bzw. für die Herstellung der einzelnen Lösungen, die zur Herstellung der kupferhaltigen Lösung vereinigt werden) Wasser oder eine wässrige Säure, wie wässrige Salzsäure (HCl), wässrige Salpetersäure (HNO₃), wässrige Schwefelsäure oder Mischungen davon, insbesondere Wasser oder wässrige Salpetersäure.

Die kupferhaltige wässrige Lösung weist vorzugsweise einen pH im Bereich von 0 bis 7, stärker bevorzugt im Bereich von 1 bis 5, besonders bevorzugt im Bereich von 2 bis 4 auf.

Als Kupferverbindungen können prinzipiell sowohl Kupfer in metallischer Form als auch vorzugsweise alle in Wasser, Säuren oder Laugen, insbesondere die in Wasser und/oder Säuren gut löslichen Verbindungen von Kupfer, insbesondere die Salze von Kupfer, ganz besonders die Nitrate; Sulfate; Halogenide, wie Chloride, Bromide und/oder Iodide; Carbonate; Oxide; Hydroxide; Hydrogencarbonate und/oder Acetate von Kupfer verwendet werden. Vorzugsweise handelt es sich bei den Kupferverbindungen um Kupfernitrat. Besonders bevorzugt wird eine wässrige, insbesondere saure, Kupfernitratlösung im erfindungsgemäßen Verfahren verwendet.

Als Zinkverbindungen können prinzipiell sowohl Kupfer in metallischer Form als auch vorzugsweise alle in Wasser, Säuren oder Laugen, insbesondere die in Wasser und/oder Säuren gut löslichen Verbindungen von Zink, insbesondere die Salze von Zink, ganz besonders die Nitrate; Sulfate; Halogenide, wie Chloride, Bromide und/oder Iodide; Carbonate; Oxide; Hydroxide; Hydrogencarbonate und/oder Acetate von Zink verwendet werden. Vorzugsweise handelt es sich bei den Zinkverbindungen um Zinknitrat. Besonders bevorzugt wird eine wässrige, insbesondere saure, Zinknitratlösung im erfindungsgemäßen Verfahren verwendet.

Als Aluminiumverbindungen können prinzipiell sowohl Aluminium in metallischer Form als auch vorzugsweise alle in Wasser, Säuren oder Laugen gut löslichen Verbindungen von Aluminium, insbesondere die Salze von Aluminium, ganz besonders die Nitrate; Sulfate; Halogenide, wie Chloride, Bromide und/oder Iodide; Oxide; Hydroxide und/oder Acetate von Aluminium verwendet werden. Vorzugsweise handelt es sich bei den Aluminiumverbindungen um Aluminiumnitrat.

Weitere bevorzugte Aluminiumverbindungen schließen Natriumaluminate und Aluminiumhydroxid-Sole und Mischungen davon ein.

Als Aluminiumhydroxid-Sol kann beispielweise ein im Handel erhältliches Produkt, z.B. ein peptisiertes Boehmit oder Pseudoboehmit verwendet werden. In diesem Fall entsteht durch das Vereinigen der Kupfer- und Zinkverbindungen mit dem Aluminiumhydroxid-Sol eine Suspension bestehend aus Kupfer-, Zink-und Aluminiumverbindungen. Alternativ kann das Aluminiumhydroxid-Sol auch dadurch erhalten werden, dass man eine wässrige, alkalische Natriumaluminatlösung (pH > 9) mit einer sauren Kupfer- und Zinksalzlösung (pH < 1) vereinigt. Im diesem Fall entsteht durch das Vereinigen der Kupfer-, Zink- und Aluminiumverbindungen eine vorgefällte, saure Suspension (pH ≤ 3,0), die die Kupfer-, Zink- und Aluminiumverbindungen enthält.

Die alkalische Lösung wird insbesondere hergestellt, indem Alkali-, Erdalkali- und/oder Ammoniumverbindungen, insbesondere Alkali-und/oder Ammoniumverbindungen, besonders bevorzugt deren Carbonate, Hydrogencarbonate und/oder Hydroxide in einem geeigneten Lösungsmittel, insbesondere Wasser gelöst werden.

Die Alkali-, Erdalkali- und Ammoniumverbindungen werden vorzugsweise ausgewählt aus der Gruppe, bestehend aus Alkalicarbonaten, wie Lithium-, Natrium-, Kalium-, Rubidium- oder Cäsium-Carbonat, Alkalihydroxiden, wie Lithium-, Natrium- oder Kalium-Hydroxid, Erdalkalicarbonaten, wie Magnesium-, Kalzium-, Strontium- oder Bariumcarbonat, Ammoniumcarbonat, Ammoniumhydroxid oder Mischungen davon. Ebenso können gleichzeitig mit oder anstelle der Carbonate die entsprechenden Hydrogencarbonate oder beliebige Mischungen aus Carbonaten und Hydrogencarbonaten verwendet werden.

Vorzugsweise verwendet man als alkalische Lösung eine wässrige Alkali- und/oder Ammoniumcarbonatlösung, eine wässrige Alkali-und/oder Ammoniumhydrogencarbonatlösung, eine wässrige Alkali-und/oder Ammoniumhydroxidlösung, insbesondere eine wässrige Natriumcarbonat-, eine wässrige Natriumhydrogencarbonatlösung und/oder eine wässrige Ammoniumhydroxidlösung (NH₃ in Wasser), besonders bevorzugt eine wässrige Natriumcarbonat- und/oder eine wässrige Natriumhydrogencarbonatlösung.

Die alkalische wässrige Lösung weist vorzugsweise einen basischen pH im Bereich von 7 bis 14, stärker bevorzugt im Bereich von 8 bis 14, besonders bevorzugt im Bereich von 10 bis 13 auf.

Durch Vereinigen der kupferhaltigen Lösung (die Kupfer, Zink und Aluminium enthält) mit der alkalischen Lösung bildet sich ein Niederschlag.

In einer Ausführungsform kann das Vereinigen erfolgen, indem die oben genannten Lösungen gleichzeitig in einen gemeinsamen Behälter, wie beispielsweise einen Fällbehälter, gegeben werden. Dabei werden die beiden Lösungen vorzugsweise kontinuierlich in das Reaktionsvolumen eines Fällmischers eingeleitet. In einer weiteren Ausführungsform kann das Vereinigen auch erfolgen, indem die eine Lösung zu der, beispielsweise in einem Behälter, wie einem Fällbehälter, vorgelegten anderen Lösung zudosiert wird. In einer bevorzugten Ausführungsform erfolgt das Vereinigen der Lösungen durch Zudosieren von einem Volumenstrom der kupferhaltigen Lösung zu der entsprechenden in einem Fällbehälter vorgelegten alkalischen Lösung.

Die kupferhaltige Lösung wird vor dem Vereinigen vorzugsweise auf eine Temperatur von 20°C oder mehr, wie zum Beispiel auf eine Temperatur im Bereich von 50°C bis 90°C, insbesondere auf etwa 65°C, erwärmt und dabei vorzugsweise gerührt.

Ebenso wird die alkalische Lösung vor dem Vereinigen vorzugsweise auf eine Temperatur von 20°C oder mehr, wie zum Beispiel auf eine Temperatur im Bereich von 50°C bis 90°C, insbesondere auf etwa 65°C, erwärmt und dabei gerührt.

In einer bevorzugten Ausführungsform werden sowohl die kupferhaltige Lösung, als auch die alkalische Lösung, auf eine Temperatur im Bereich von 50°C bis 90°C, insbesondere auf etwa 65°C erwärmt und dabei gerührt.

Beim Vereinigen von Lösungen der oben genannten Lösungspaare bildet sich ein Niederschlag in der Mischung (im Folgenden auch als niederschlagshaltige Lösungsmischung bezeichnet). Das Vereinigen der Lösungen erfolgt in der Regel in einem gerührten Behälter. Der Behälter wird vorzugsweise mit einem Schrägblattrührer, Propellerrührer oder sonstigen handelsüblichen Rührern gerührt.

Die niederschlagshaltige Lösungsmischung wird vorzugsweise auf einer Temperatur von 20°C oder mehr und insbesondere auf einer Temperatur im Bereich von 50°C bis 90°C, bevorzugt auf etwa 65°C, gehalten. In einer besonders bevorzugten Ausführungsform der Erfindung wird die niederschlagshaltige Lösungsmischung mindestens 30 Minuten, vorzugsweise 1 h bis 36 h, insbesondere etwa 2 h, auf einer Temperatur im Bereich von 50°C bis 90°C, vorzugsweise auf einer Temperatur von etwa 65°C, gehalten, um die Niederschlagsbildung gegebenenfalls zu vervollständigen bzw. durch Alterung die Kristallinität des Niederschlages zu erhöhen.

Bis zur Vervollständigung der Niederschlagsbildung wird der pH-Wert der niederschlagshaltigen Lösungsmischung üblicherweise durch dem Fachmann bekannte Verfahren konstant gehalten. Beispielsweise kann die Zudosierungsgeschwindigkeit von Lösungen so gewählt werden, dass sich ein bestimmter pH-Wert in der niederschlagshaltigen Lösungsmischung einstellt. Vorzugsweise liegt der pH-Wert der niederschlagshaltigen Lösungsmischung im Bereich von 5,0 bis 8,5, insbesondere im Bereich von 6,0 bis 7,5, bevorzugt bei etwa 6,5.

Der in Schritt (a) erhaltene Niederschlag wird in Schritt (b) vorzugsweise durch Filtration abgetrennt. Alternativ dazu kann der Niederschlag auch durch Dekantieren oder Zentrifugieren abgetrennt werden.

Der abgetrennte Niederschlag wird gegebenenfalls einem oder mehreren Waschschritten unterzogen und anschließend gegebenenfalls getrocknet. Durch Abtrennen, gegebenenfalls Waschen und gegebenenfalls Trocknen des Niederschlags wird ein fester Katalysatorvorläufer gebildet.

Das Waschen des Niederschlags kann beispielweise dadurch erfolgen, dass die niederschlagshaltige Lösungsmischung zuerst durch Verwendung einer Filterpresse vom Niederschlag abgetrennt und das Material anschließend in der Filterpresse mit Wasser durchströmt und dadurch gewaschen werden. Alternativ kann der abgetrennte Niederschlag nach dem Abtrennen der niederschlagshaltigen Lösungsmischung durch Filtrieren, Dekantieren oder Zentrifugieren in einem Behälter aufgeschlämmt und anschließend erneut mit Hilfe einer Filterpresse, einer Zentrifuge oder eines Dekanters von der flüssigen Phase getrennt werden. Dieser Vorgang wird in der Regel ein oder mehrere Male bis zum Erreichen eines bestimmten Gehalts an Natriumionen im Filterrückstand, d.h. im Filterkuchen, durchgeführt. Die Bestimmung des Gehalts an Natriumionen kann durch Atomabsorptionsspektroskopie (AAS) erfolgen. Der Gehalt an Natrium im Filterkuchen beträgt nach dem letzten Waschgang vorzugsweise 500 ppm oder weniger, stärker bevorzugt weniger 400 ppm oder weniger, insbesondere 350 ppm oder weniger. Alternativ kann das Waschen auch bis zum Erreichen einer bestimmten Leitfähigkeit des Filtrats durchgeführt werden. Dabei korreliert die Leitfähigkeit in der Regel mit der Konzentration an Natriumionen. Die Leitfähigkeit des Filtrats des letzten Waschvorgangs beträgt vorzugsweise 0,5 mS/cm oder weniger, insbesondere 0,2 mS/cm oder weniger. Die Leitfähigkeit wird nach DIN 38404, Teil 8 bestimmt.

Der abgetrennte und gegebenenfalls gewaschene Niederschlag wird anschließend vorzugsweise einer Trocknung unterzogen. Die Trocknung erfolgt durch Erwärmen des Niederschlags auf eine Temperatur im Bereich von 75°C bis 130°C, vorzugsweise in einem Bereich von 105°C bis 115°C.

In einer besonders bevorzugten Ausführungsform erfolgt die Trocknung durch Sprühtrocknung. Dazu wird aus dem abgetrennten Niederschlag, wie einem Filterkuchen, mit Wasser eine Suspension mit einem Feststoffgehalt von 10 bis 40 Gew.% hergestellt. Diese Suspension wird vorzugsweise anschließend über eine Düse in einen Sprühtrockner eindosiert. Die Temperatur im Sprühtrockner liegt während der Trocknung vorzugsweise in einem Bereich von 75°C bis 130°C, insbesondere in einem Bereich von 105°C bis 115°C. Die für die Trocknung charakteristische Austrittstemperatur liegt vorzugsweise im Bereich von 105°C bis 115°C und wird üblicherweise durch Parameter, wie eingesprühte Menge an Suspension, der Feststoffgehalt der Suspension (und damit der Menge an Wasser, welche verdampft werden muss) bzw. Temperatur im Sprühtrockner gesteuert. Aus der Behandlung des Materials mit dem Sprühtrockner resultiert insbesondere ein trockenes Pulver.

Ein Teil des in Schritt (b) erhaltenen festen Katalysatorvorläufers wird in Schritt (c) einer thermischen Behandlung unterzogen, wobei ein Mischoxid erhalten wird.

Bei der thermischen Behandlung werden die Metalle durch Zersetzung der Carbonate aus dem gegebenenfalls sprühgetrockneten Vorläufermaterial (zumindest teilweise) in die entsprechenden Oxide überführt. Die spezifische BET-Oberfläche beträgt im Bereich von 80 m²/g bis 140 m²/g, bevorzugt im Bereich von 85 m²/g bis 120 m²/g, besonders bevorzugt im Bereich von 90 m²/g bis 110 m²/g. Diese kann durch die Temperatur und Dauer der thermischen Behandlung (Kalzinierung) gesteuert werden. Bevorzugte Kalzinierungstemperaturen liegen im Bereich von 200°C bis 600°C, bevorzugt im Bereich von 270°C bis 550°C, und besonders bevorzugt im Bereich von 450°C und 500°C. Die Dauer der thermischen Behandlung beträgt vorzugsweise von 1 h bis 5 h, bevorzugt von 2,5 h bis 4 h, besonders bevorzugt etwa 3 h.

In einer besonders bevorzugten Ausführungsform erfolgt die thermische Behandlung über einen Zeitraum von 2,5 h bis 4 h bei einer Temperatur im Bereich von 450°C und 500°C.

Die thermische Behandlung kann unter Luft, unter Sauerstoff oder unter Schutzgas, wie beispielsweise Argon oder Stickstoff, oder Mischungen davon durchgeführt werden. Die thermische Behandlung kann diskontinuierlich, z.B. in einem Hordenofen oder kontinuierlich, z.B. im Drehrohrofen durchgeführt werden.

In Schritt (d) wird das in Schritt (c) erhaltene Mischoxid mit einem Teil des in Schritt (b) erhaltenen festen Katalysatorvorläufers (der keiner thermischen Behandlung unterzogen wurde) gemischt. Das Gewichtsverhältnis von festem (nicht thermisch behandeltem) Katalysatorvorläufer zu thermisch behandeltem Katalysatorvorläufer (Mischoxid) liegt im Bereich von 2 : 98 bis 20 : 80, vorzugsweise im Bereich von 5 : 95 bis 15 : 85, bevorzugter im Bereich von 10 : 90 bis 15 : 85.

Die in Schritt (d) erhaltene Mischung wird anschließend (vorzugsweise unter Zugabe von Schmiermittel) in einem Schritt (e) tablettiert.

Die Tablettierung wird vorzugsweise mit einer Tablettenpresse, wie beispielsweise einer Korsch-Tablettenpresse, durchgeführt. Durch das

Tablettieren können Tabletten mit einem Durchmesser d von 1 mm bis 10 mm, bevorzugt von 1,5 mm bis 8 mm und besonders bevorzugt von 4 mm bis 6 mm und einer Höhe h von 1 mm bis 10 mm, bevorzugt von 1,5 mm bis 8 mm und besonders bevorzugt von 3 mm bis 4 mm erhalten werden.

Die Tablettierung erfolgt vorzugsweise unter Zugabe eines Schmiermittels wie Graphit, Ölen oder Stearaten, insbesondere Graphit. Dabei wird das (in Schritt (d)) erhaltene Gemisch aus thermisch behandeltem Mischoxid (aus Schritt (c)) und festem Katalysatorvorläufer (aus Schritt (b)) mit Schmiermitteln, insbesondere Graphit, gemischt, gegebenenfalls kompaktiert und/oder granuliert und dann in Schritt (e) tablettiert. Vorzugsweise wird das Schmiermittel vor dem Tablettieren in einer Menge im Bereich von 0,1 bis 5 Gew.%, bezogen auf das Gesamtgewicht der zu tablettierenden Masse zugegeben. Stärker bevorzugt wird das Schmiermittel in einer Menge im Bereich von 0,5 bis 5 Gew.%, besonders bevorzugt in einer Menge im Bereich von 1 bis 3 Gew.%, vorzugsweise von etwa 2 Gew.% bezogen auf das Gesamtgewicht der zu tablettierenden Masse zugegeben.

Der nach der Tablettierung erhaltene Katalysatorformkörper weist eine auf das Tablettengewicht bezogene Seitendruckfestigkeit, gemessen gemäß DIN EN 1094-5, im Bereich von 550 N/g oder mehr, bevorzugt im Bereich von 600 N/g bis 1300 N/g, insbesondere im Bereich von 600 bis 900 N/g auf.

In einer bevorzugten Ausführungsform besitzen die tablettierten Katalysatorformkörper einen Durchmesser d im Bereich von 4 mm bis 6 mm und eine Höhe h im Bereich von 3 mm bis 4 mm, und weisen eine Seitendruckfestigkeit, bezogen auf das Tablettengewicht, im Bereich von 600 bis 900 N/g auf.

Die tablettierten Katalysatorformkörper weisen vorzugsweise einen Glühverlust von 7,5 Gew.% oder weniger, bevorzugt 5,5 Gew.% oder weniger, insbesondere im Bereich von 0,1 bis 4,0 Gew.% auf.

In einer weiteren bevorzugten Ausführungsform weist der erhaltene Katalysatorformkörper eine auf das Tablettengewicht bezogene Seitendruckfestigkeit, gemessen gemäß DIN EN 1094-5, im Bereich von 600 bis 900 N/g und einen Glühverlust im Bereich von 0,1 bis 4,0 Gew.% auf.

In einer weiteren Ausführungsform wird die in Schritt (e) erhaltene tablettierte Mischung in einem weiteren Schritt (f) reduziert.

Die Reduktion erfolgt vorzugsweise durch Erhitzen des tablettierten Katalysatorformkörpers in einer reduzierenden Atmosphäre. Insbesondere handelt es sich bei der reduzierenden Atmosphäre um Wasserstoff. Die Reduktion erfolgt beispielsweise bei einer Temperatur im Bereich von 150°C bis 450°C, insbesondere im Bereich von 180°C bis 300°C, vorzugsweise im Bereich von 190°C bis 290°C, besonders bevorzugt bei etwa 250°C.

Die Reduktion erfolgt beispielsweise in Abhängigkeit von der zu reduzierenden Katalysatormenge über einen Zeitraum von 1 Stunde (für beispielsweise 500 g) bis 10 Tage (für beispielsweise 60 Tonnen), insbesondere über einen Zeitraum von 2 Stunden bis 120 Stunden, vorzugsweise über einen Zeitraum von 24 bis 48 Stunden reduziert. Katalysatormengen im Produktionsmaßstab (beispielsweise im Bereich von 1 bis 60 Tonnen) werden vorzugsweise über einen Zeitraum von 3 bis 8 Tagen reduziert. In einer bevorzugten Ausführungsform erfolgt die Reduktion bei einer Temperatur im Bereich von 190°C bis 210°.

Vorzugsweise werden die Katalysatorformkörper nach der Reduktion nass oder trocken stabilisiert. Bei der Nassstabilisierung werden die Formkörper mit Flüssigkeit überschichtet, um den Kontakt mit Sauerstoff möglichst zu vermeiden. Geeignete Flüssigkeiten schließen organische Flüssigkeiten und Wasser, vorzugsweise organische Flüssigkeiten ein. Bevorzugte organische Flüssigkeiten sind solche, die bei 20°C einen Dampfdruck von 0,5 hPa oder weniger aufweisen. Beispiele geeigneter organischer Flüssigkeiten sind Iso-Decanol, Fettalkohole, wie z.B. Nafol® der Firma Sasol, Hexadecan, 2-Ethylhexanol, Propylenglycol und Mischungen davon, insbesondere Iso-Decanol.

Bei der Trockenstabilisierung wird in den Reduktionsreaktor ein Gemisch aus Sauerstoff oder einem sauerstoffhaltigen Gas, vorzugsweise Luft, und einem inerten Gas, wie Argon oder Stickstoff zudosiert. Die Konzentration an Sauerstoff in dem Gemisch wird vorzugsweise von etwa 0,04 Vol.% auf etwa 21 Vol.% erhöht. Beispielsweise kann ein Gemisch aus Luft und Inertgas zudosiert werden, wobei das Verhältnis von Luft zu Inertgas anfangs etwa 0,2 Vol.% Luft zu 99,8 Vol.% Inertgas beträgt. Das Verhältnis von Luft zu Inertgas wird dann allmählich erhöht (z.B. kontinuierlich oder schrittweise) bis letztendlich beispielsweise 100 Vol.% Luft zudosiert wird (was einer Sauerstoffkonzentration von etwa 21 Vol.% entspricht). Ohne an eine Theorie gebunden zu sein, wird vermutet, dass durch die Zudosierung von Luft oder Sauerstoff eine dünne Oxidschicht mit einer Dicke von beispielsweise 0,5 nm bis 50 nm, vorzugsweise 1 nm bis 20 nm, insbesondere 1 nm bis 10 nm an der Oberfläche des Katalysators entsteht, welche den Katalysator vor weiterer Oxidation schützt. Bei der Trockenstabilisierung beträgt die Reaktortemperatur vorzugsweise 100°C oder weniger, noch bevorzugter 20°C bis 70°C und besonders bevorzugt 30°C bis 50°C. Nach dieser Stabilisierung ist der Katalysator transportfähig und kann zum Anwender/Anlagenbetreiber transportiert werden.

Die Volumenschrumpfung der Tabletten nach Reduktion und Passivierung wird durch Vermessen der Tablettenabmessungen (Durchmesser und Höhe) einer repräsentativen Anzahl von 20 Tabletten bestimmt.

Die nach dem erfindungsgemäßen Verfahren hergestellten Katalysatorformkörper zeigen in einer besonderen Ausführungsform eine Volumenschrumpfung durch Reduktion von 8 % oder weniger, bevorzugt von 6 % oder weniger, insbesondere von 5 % oder weniger.

Das Cu/Zn-Atomverhältnis im Katalysatorformkörper kann in weiten Grenzen variieren, wird aber bevorzugt an das von herkömmlichen Methanolsynthesekatalysatoren angepasst. Das Cu/Zn-Atomverhältnis im Katalysatorformkörper beträgt bevorzugt 15:85 bis 85:15, besonders bevorzugt 60:40 bis 75:25. Das Zn/Al-Atomverhältnis beträgt bevorzugt 60:40 bis 80:20, besonders bevorzugt 70:30 bis 80:20.

In einer bevorzugten Ausführungsform beträgt das Cu/Zn-Atomverhältnis 15:85 bis 85:15 und das Zn/Al-Atomverhältnis 60:40 bis 80:20. In einer besonders bevorzugten Ausführungsform beträgt das Cu/Zn-Atomverhältnis 60:40 bis 75:25 und das Zn/Al-Atomverhältnis 70:30 bis 80:20.

Der erfindungsgemäße kupferhaltige Katalysatorformkörper eignet sich für den großtechnischen Einsatz. Der Begriff "Katalysatorformkörper" kann in der vorliegenden Erfindung austauschbar mit dem Begriff "Katalysator" verwendet werden, insbesondere wenn auf die Funktion als solches abgestellt wird.

Gegenstand der Erfindung ist auch die Verwendung des vorstehend beschriebenen Katalysators zur Synthese von Methanol aus Synthesegas, das heißt aus Gas, das CO₂, CO und H₂ enthält. Das Synthesegas besteht üblicherweise aus 5 Vol.% bis 25 Vol.% Kohlenmonoxid, 6 Vol.% bis 12 Vol.% Kohlenstoffdioxid, 10 Vol.% bis 30 Vol.% inerte Gase, wie z.B. Stickstoff und/oder Methan, und als Rest Wasserstoff.

Die Methanolsynthese wird üblicherweise bei einer Temperatur von im Bereich von 200°C bis 300°C, vorzugsweise im Bereich von 210°C bis 280°C, bei einem Druck im Bereich von 40 bar bis 150 bar, vorzugsweise im Bereich von 60 bar bis 100 bar, und einer Raumgeschwindigkeit im Bereich von 2.000 bis 22.000 h⁻¹ durchgeführt. Die Raumgeschwindigkeit ist definiert als Quotient aus dem Volumenstrom des Synthesegases zum räumlichen Volumen des Katalysators, z.B. eines Katalysatorbetts, bezogen auf die Zeiteinheit von einer Stunde.

Der erfindungsgemäße kupferhaltige Katalysator eignet sich ferner für die Konvertierung von CO zu CO₂, insbesondere die Tieftemperatur-Konvertierung von CO zu CO₂. Die Konvertierung von CO zu CO₂ erfolgt gemäß der folgenden Reaktionsgleichung:

CO + H₂O <=> H₂ + CO₂

Die Tieftemperatur-Konvertierung wird üblicherweise bei einer Temperatur im Bereich von 170°C bis 270°C, bevorzugt im Bereich von 190°C bis 240°C durchgeführt. Die Tieftemperatur-Konvertierung erfolgt üblicherweise bei einem Druck im Bereich von 1 bar bis 40 bar, bevorzugt im Bereich von 10 bar bis 35 bar. In einer bevorzugten Ausführungsform wird die Tieftemperatur-Konvertierung bei einer Temperatur im Bereich von 170°C bis 270°C und einem Druck im Bereich von 1 bar bis 40 bar, insbesondere bei einer Temperatur im Bereich von 190°C bis 240°C und einem Druck im Bereich von 10 bar bis 35 bar durchgeführt.

### Bestimmung physikalischer Parameter

Die in der vorliegenden Erfindung angeführten physikalischen Parameter werden, soweit nicht anders angegeben, wie nachfolgend beschrieben bestimmt:
Bestimmung der BET-Oberfläche: Die BET-Oberfläche wird nach der Stickstoff-Einpunktmethode am pulverförmigen Katalysator sowie an Tabletten mit einem Durchmesser von 6 mm und einer Höhe von 4 mm entsprechend DIN 66132 bestimmt.

Bestimmung des Glühverlustes: Die Bestimmung des Glühverlustes erfolgt ausgehend vom Pulver. Zur Bestimmung des Glühverlustes der Tabletten werden diese zuvor zu Pulver gemahlen. Die zu bestimmende Probe wird in einen zuvor bei 600°C für 3 h in einem Muffelofen ausgeglühten Prozellantiegel eingewogen. Anschließend wird die in den ausgeglühten und austarierten Prozellantiegel eingewogene Probe bei 600°C für 3 h in einem Muffelofen thermisch behandelt, in einen Exsikkator überführt und auf Raumtemperatur abgekühlt. Der abgekühlte Tiegel wird erneut gewogen. Aus der Massendifferenz wird der Glühverlust bei 600°C ermittelt.

Bestimmung der Seitendruckfestigkeit: Die Seitendruckfestigkeit (SDF) der Formköper/Tabletten wird nach DIN EN 1094-5, Ausgabe 1995-09, feuerfeste Ergebnisse für Isolationszwecke - Teil 5: "Bestimmung der Kaltdruckfestigkeit geformter Erzeugnisse" bestimmt. Die Bestimmung wird mit einem handelsüblichen Gerät, wie z.B. Modell SCHLEUNIGER 6-D oder ERWEKA TBH 310 MD, nach den Angaben des Geräteherstellers durchgeführt. Typischerweise wird für eine Vielzahl von Tabletten (z.B. von 10 bis 100, vorzugsweise 10 bis 30, beispielsweise 20 Tabletten) die am Zylindermantel der Tabletten anliegenden Pressdrücke beim Berstvorgang bestimmt. Aus den erhaltenen Werten (in N) wird der arithmetische Mittelwert gebildet. Die auf das Tablettengewicht bezogene Seitendruckfestigkeit (in N/g) ergibt sich durch eine Normierung des für die Seitendruckfestigkeit erhaltenen arithmetischen Mittelwerts auf das arithmetische gemittelte Tablettengewicht.

Bestimmung des Porenvolumens der Tabletten: Das Porenvolumen wird nach der Quecksilber-Intrusionsmethode entsprechend DIN 66133 am pulverförmigen oxidischen Katalysator sowie an Tabletten durchgeführt.

### Beispiele

Anhand der folgenden, nicht beschränkenden Beispiele wird die Erfindung näher erläutert. Wenngleich diese Beispiele spezielle Ausführungsformen der Erfindung beschreiben, dienen sie nur zur Veranschaulichung der Erfindung und sollen nicht als die Erfindung in irgendeiner Weise einschränkend aufgefasst werden. Wie der Fachmann weiß, können zahlreiche Änderungen daran durchgeführt werden, ohne vom Schutzumfang der Erfindung, wie er durch die beigefügten Patentansprüche definiert wird, abzuweichen.

### Herstellung der Katalysatoren

Zur Herstellung der Katalysatoren wurde eine 14 gew.%ige wässrige Sodalösung hergestellt und auf 50°C erwärmt. In einem zweiten Behälter wurde bei 50°C 820 g Kupfernitrat, 120 g Zinkoxid und 260 g Aluminiumnitrat in 900 g Wasser und 270 g 68 gew.%iger HNO₃ gelöst. Die Nitratlösung und die Sodalösung wurden bei einer Temperatur von 65°C gleichzeitig unter Konstanthaltung des pH-Wertes von 6,5 zusammengeführt (Fällung). Die Suspension wurde kontinuierlich vom Fällungsbehälter in einen Alterungsbehälter gepumpt. Nach Beendigung der Fällung wurde die Suspension für mindestens 120 Minuten bei 70°C gealtert. Die Farbe änderte sich von hellblau (Beginn der Alterung) zu grün (Ende der Alterung). Nach der Alterung wurde die Suspension filtriert und der Filterkuchen gewaschen bis der Natriumgehalt des Filterkuchens, bestimmt durch Atomabsorptionsspektroskopie, weniger als 350 ppm betrug. Der Filterkuchen wurde durch Zugabe von Wasser bis auf eine Oxidkonzentration von 10 Gew.% aufgeschlämmt und im Sprühtrockner bei einer Eintrittstemperatur von 275°C bis 270°C und einer Austrittstemperatur von 105°C bis 115°C zum Erhalt eines festen Katalysatorvorläufers getrocknet. Der erhaltene feste Katalysatorvorläufers wurde für die Herstellung der nachfolgend beschriebenen Katalysatorformkörper eingesetzt.

Zur analytischen Bestimmung der Zusammensetzung wurde ein Teil des festen Katalysatorvorläufers 2 h bei 330°C kalziniert. Die chemische Zusammensetzung betrug (Angaben in Gew.%): 64,0% CuO, 27,8% ZnO, 8,2% Al₂O₃. Der feste Katalysatorvorläufer wurde anschließend bei verschiedenen Temperaturen thermisch behandelt (Schritt (c)) und im Fall der erfindungsgemäßen Katalysatorformkörper im angegebenen Verhältnis mit nicht thermisch behandeltem festem Katalysatorvorläufermaterial gemischt (Schritt (d)). Schließlich wurde die Mischung unter Zugabe von jeweils 2 Gew.% Graphit, bezogen auf das Gewicht der Mischung, zu Tabletten mit einem Durchmesser von 6 mm und einer Höhe von 4 mm tablettiert (Schritt (e)).

**Vergleichskatalysator 1 (Ex11519.01):** Die thermische Behandlung erfolgte in einem Muffelofen bei 400°C für 3 h. Das erhaltene Pulver wies eine BET-Oberfläche von 119 m²/g und einen Glühverlust von 11,9 Gew.% auf. Anschließend wurden 100 g des Pulvers mit 2 g Graphit gemischt und das Gemisch zu Formkörpern mit einem Durchmesser von 6 mm und einer Höhe von 4 mm tablettiert. Die auf das Tablettengewicht bezogene Seitendruckfestigkeit betrug 1021,5 N/g.

**Vergleichskatalysator 2 (Ex11519.02):** Die thermische Behandlung erfolgte in einem Muffelofen bei 430°C für 3 h. Das erhaltene Pulver wies eine BET-Oberfläche von 117 m²/g und einen Glühverlust von 9,0 Gew.% auf. Anschließend wurden 100 g des Pulvers mit 2 g Graphit gemischt und das Gemisch zu Formkörpern mit einem Durchmesser von 6 mm und einer Höhe von 4 mm tablettiert. Die auf das Tablettengewicht bezogene Seitendruckfestigkeit betrug 1020,4 N/g.

**Katalysator 1 (Ex11519.04):** Die thermische Behandlung erfolgte in einem Muffelofen bei 460°C für 3 h. Das erhaltene Pulver wies eine BET-Oberfläche von 114 m²/g und einen Glühverlust von 4,4 Gew.% auf. Anschließend wurden 95 g des Pulvers mit 5 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 868,2 N/g.

**Katalysator 2 (Ex11519.05):** Die thermische Behandlung erfolgte in einem Muffelofen bei 500°C für 3 h. Das erhaltene Pulver wies eine BET-Oberfläche von 99 m²/g und einen Glühverlust von <0,5 Gew.% auf. Anschließend wurden 95 g des Pulvers mit 5 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 792,3 N/g.

**Katalysator 3 (Ex11519.06):** Die thermische Behandlung erfolgte in einem Muffelofen bei 500°C für 3 h. Das erhaltene Pulver wies eine BET-Oberfläche von 99 m²/g und einen Glühverlust von <0,5 Gew.% auf. Anschließend wurden 90 g des Pulvers mit 10 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 894,7 N/g.

**Katalysator 4 (Ex11519.07):** Die thermische Behandlung erfolgte in einem Muffelofen bei 500°C für 3 h. Das erhaltene Pulver wies eine BET-Oberfläche von 99 m²/g und einen Glühverlust von <0,5 Gew.% auf. Anschließend wurden 85 g des Pulvers mit 15 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 899,1 N/g.

**Katalysator 5 (Ex11519.08):** Die thermische Behandlung erfolgte in einem Muffelofen bei 550°C für 3 h. Das erhaltene Pulver wies eine BET-Oberfläche von 92 m²/g und einen Glühverlust von <0,5 Gew.% auf. Anschließend wurden 95 g des Pulvers mit 5 g nicht thermisch behandeltem Material (erhalten aus Schritt (b)) gemischt und das Gemisch unter Zugabe von 2 g Graphit zu Formköpern mit einem Durchmesser von 6 mm und einer Höhe von 4 mm tablettiert. Die auf das Tablettengewicht bezogene Seitendruckfestigkeit betrug 570,1 N/g.

**Katalysator 6 (Ex11519.09):** Die thermische Behandlung erfolgte in einem Muffelofen bei 550°C für 3 h. Das erhaltene Pulver wies eine BET-Oberfläche von 92 m²/g und einen Glühverlust von <0,5 Gew.% auf. Anschließend wurden 90 g des Pulvers mit 10 g nicht thermisch behandeltem Material (erhalten aus Schritt (b)) gemischt und das Gemisch unter Zugabe von 2 g Graphit zu Tabletten mit einem Durchmesser von 6 mm und einer Höhe von 4 mm tablettiert. Die auf das Tablettengewicht bezogene Seitendruckfestigkeit betrug 620,1 N/g.

**Katalysator 7 (Ex11519.10):** Die thermische Behandlung erfolgte in einem Muffelofen bei 550°C für 3 h. Das erhaltene Pulver wies eine BET-Oberfläche von 92 m²/g und einen Glühverlust von <0,5 Gew.% auf. Anschließend wurden 85 g des Pulvers mit 15 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 618,4 N/g.

**Katalysator 8 (Ex11519.11):** Die thermische Behandlung erfolgte in einem Muffelofen bei 550°C für 3 h. Das erhaltene Pulver wies eine BET-Oberfläche von 92 m²/g und einen Glühverlust von <0,5 Gew.% auf. Anschließend wurden 80 g des Pulvers mit 20 g nicht thermisch behandeltem Material (erhalten aus Schritt (b)) gemischt und das Gemisch unter Zugabe von 2 g Graphit zu Formkörpern mit einem Durchmesser von 6 mm und einer Höhe von 4 mm tablettiert. Die auf das Tablettengewicht bezogene Seitendruckfestigkeit betrug 696,4 N/g.

**Tabelle 1: Physikalische Eigenschaften der erhaltenen oxidischen Katalysatoren nach Direkttablettierung zu Volltabletten mit den Maßen d = 6 mm und h = 4 mm.**

| | **BET [m²/g]** | **GV [Gew. %]** | **SDF [N]** | **SDF [N/g]** | **PV [mm³ /g]** |
|---|---|---|---|---|---|
| **Vergleichskatalysator 1** | 92 | 14,0 | 244,5 | 1021,5 | 166,5 |
| **Vergleichskatalysator 2** | 97 | 8,9 | 248,7 | 1020,4 | 192,9 |
| **Katalysator 1** | 85 | 7,2 | 216,6 | 868,2 | 186,6 |
| **Katalysator 2** | 84 | 4,6 | 194,8 | 792,3 | 202,8 |
| **Katalysator 3** | 83 | 5,4 | 218,1 | 894,7 | 208,5 |
| **Katalysator 4** | 91 | 6,5 | 224,9 | 899,1 | 180,3 |
| **Katalysator 5** | 82 | 0,7 | 126,8 | 570,1 | 289,6 |
| **Katalysator 6** | 83 | 1,1 | 142,7 | 620, 1 | 222,9 |
| **Katalysator 7** | 87 | 1,9 | 140,2 | 618,4 | 221,4 |
| **Katalysator 8** | 90 | 2,3 | 156,1 | 696,4 | 241,3 |

### Aktivierung der Katalysatoren

Anschließend wurden die erhaltenden Katalysatoren der Vergleichskatalysatoren 1 und 2 und der erfindungsgemäßen Katalysatoren 1 bis 4 in Tablettenform aktiviert, d.h. im Wasserstoffstrom reduziert. Ein Menge von jeweils 200 ml der getesteten Katalysatortabletten wurde drucklos, d.h. bei Normaldruck (etwa 1,01325 bar) in einem Reaktionsrohr reduziert, wobei die

Tabletten im strömenden Reduktionsgas (900 L_{Gas}/L_{Katalysator}/h) bestehend aus 2 Vol.% Wasserstoff und etwa 98 Vol.% Stickstoff temperaturprogrammiert auf 240°C aufgeheizt wurden. Danach wurde die Temperatur auf 250°C erhöht und in reinem Wasserstoff (400 L_{Gas}/L_{Katalysator}/h) die Reduktion komplettiert. Unter Inertgas (Stickstoff) wurde auf Raumtemperatur abgekühlt und die Katalysatoren wurden in verdünnter Sauerstoffatmosphäre (0,5 Vol.% Sauerstoff und etwa 99,5 Vol.% Stickstoff) bei maximal 30°C an der Oberfläche passiviert.

Die Volumenschrumpfung der Tabletten nach Reduktion und Passivierung wurde hierbei durch Vermessen der Tablettenabmessungen (Durchmesser und Höhe) einer repräsentativen Anzahl von 20 Tabletten bestimmt.

Des Weiteren wurde die Höhe der Katalysatorschüttung im Reduktionsreaktor vor sowie nach der Reduktion gemessen und aus deren Differenz der Schwund der Katalysatorschüttung bestimmt. Beide Methoden (Bestimmung der Tablettenschrumpfung sowie Bestimmung der Schrumpfung der Katalysatorschüttung) eignen sich gleichermaßen zur Quantifizierung der Schrumpfung. In der nachfolgenden Tabelle 2 sind die für die 6 verschiedenen Katalysatorformkörper erhaltenen Werte für die durchschnittliche Volumenschrumpfung der Tabletten sowie für die durchschnittliche Volumenschrumpfung der Katalysatorschüttung angegeben:

**Tabelle 2: Schrumpfung der erhaltenen Katalysatoren nach Reduktion im Wasserstoffstrom.**

| | **Volumenschrumpfung Tabletten [%]** | **Volumenschrumpfung Katalysatorschüttung [%]** |
|---|---|---|
| **Vergleichskatalysator 1** | -10,8 | -11,2 |
| **Vergleichskatalysator 2** | -9,3 | -8,2 |
| **Katalysator 1** | -5,9 | -5,3 |
| **Katalysator 2** | -0,6 | -2,4 |
| **Katalysator 3** | -2,2 | -4,1 |
| **Katalysator 4** | -1,9 | -1,1 |

Aus Tabelle 1 ergibt sich, dass die Vergleichskatalysatoren im Vergleich zu den erfindungsgemäßen Katalysatoren im nicht reduzierten Zustand einen etwas höheren Glühverlust (bzw. eine etwas höhere BET-Oberfläche) aufweisen. Der geringere Glühverlust der erfindungsgemäßen Katalysatorformköper korreliert mit einer geringeren auf das Tablettengewicht bezogenen Seitendruckfestigkeit.

Allerdings zeigen die Vergleichskatalysatoren eine deutlich größere Volumenschrumpfung nach der Reduktion im Wasserstoffstrom (siehe Tabelle 2). Während bei den Vergleichskatalysatoren Schrumpfungen im Bereich von etwa 10 % beobachtet werden, zeigen die erfindungsgemäßen Katalysatorformkörper eine deutlich verringerte Schrumpfung von etwa 6 % bis weniger als 1 %. Die verringerte Schrumpfung bei gleichzeitig guter mechanischer Festigkeit ermöglicht eine verbesserte Ausnutzung des Reaktorvolumens und damit eine wirtschaftlichere Nutzung der Katalysatorformkörper.

Zusammenfassend lässt sich also festhalten, dass sich die durch das erfindungsgemäße Verfahren erhältlichen Katalysatorformkörper durch eine hohe mechanische Festigkeit in Verbindung mit einer stark verringerten Schrumpfung nach Reduktion auszeichnen.

## Patentansprüche

1. Verfahren zur Herstellung eines Katalysatorformkörpers, enthaltend Kupfer, Zink und Aluminium, umfassend die folgenden Schritte:
(a) Vereinigen einer alkalischen Lösung, insbesondere eines carbonathaltigen Fällungsmittels,
mit einer kupferhaltigen Lösung, die erhältlich ist durch Lösen und/oder Suspendieren von einer Kupferverbindung, einer Zinkverbindung und einer Aluminiumverbindung,
zum Erhalt eines Niederschlags;
(b) Abtrennen, gegebenenfalls Waschen und/oder gegebenenfalls Trocknen des Niederschlags zum Erhalt eines festen Katalysatorvorläufers;
(c) thermische Behandlung von in Schritt (b) erhaltenem festen Katalysatorvorläufer bei einer Temperatur im Bereich von 200°C bis 600°C, bevorzugt im Bereich von 270°C bis 550°C, und besonders bevorzugt im Bereich von 450°C und 500°C, zum Erhalt eines Mischoxids, mit einer BET-Oberfläche gemäß DIN 66132 im Bereich von 80 m²/g bis 140 m²/g, bevorzugt im Bereich von 85 m²/g bis 120 m²/g, besonders bevorzugt im Bereich von 90 m²/g bis 110 m²/g;
(d) Mischen von in Schritt (b) erhaltenem festen Katalysatorvorläufer mit in Schritt (c) erhaltenem Mischoxid in einem Gewichtsverhältnis von festem Katalysatorvorläufer zu Mischoxid im Bereich von 2 : 98 bis 20 : 80, vorzugsweise im Bereich von 5 : 95 bis 15 : 85, bevorzugter im Bereich von 10 : 90 bis 15 : 85 zum Erhalt einer Mischung; und
(e) Tablettieren der in Schritt (d) erhaltenen Mischung,
wobei der Katalysatorformkörper eine auf das Tablettengewicht bezogene Seitendruckfestigkeit, gemessen gemäß DIN EN 1094-5, von 500 N/g oder mehr, bevorzugt von 600 N/g oder mehr, insbesondere im Bereich von 600 bis 900 N/g aufweist.

2. Verfahren gemäß Anspruch 1, wobei der kupferhaltige Katalysatorformkörper einen Glühverlust , bestimmt nach der Methode beschrieben auf Seiten 16 und 17 der Beschreibung, von 7,5 Gew.% oder weniger, bevorzugt 5,5 Gew.% oder weniger, insbesondere im Bereich von 0,1 bis 4,0 Gew.% aufweist.

3. Verfahren gemäß Anspruch 1 oder 2, wobei die kupferhaltige Lösung in Schritt (a) ein Aluminiumhydroxid-Sol umfasst.

4. Verfahren gemäß einem der Ansprüche 1 bis 3, wobei die kupferhaltige Lösung einen pH-Wert von ≤ 3,0 aufweist.

5. Katalysatorformkörper, erhältlich nach einem Verfahren gemäß einem der Ansprüche 1 bis 4.

6. Katalysatorformkörper gemäß Anspruch 5, der eine auf das Tablettengewicht bezogene Seitendruckfestigkeit, gemessen gemäß DIN EN 1094-5, im Bereich von 600 N/g bis 1300 N/g, insbesondere im Bereich von 600 bis 900 N/g aufweist.

7. Katalysatorformkörper gemäß Anspruch 5 oder 6, der eine Volumenschrumpfung durch Reduktion, vorzugsweise durch Reduktion in einem Wasserstoffstrom, von 6% oder weniger, bevorzugt von 5% oder weniger aufweist.

8. Verfahren gemäß einem der Ansprüche 1 bis 4, umfassend den folgenden weiteren Schritt:
(f) Reduzieren der in Schritt (e) erhaltenen tablettierten Mischung.

9. Verfahren gemäß Anspruch 8, wobei das Reduzieren mit Wasserstoff erfolgt.

10. Katalysatorformkörper, erhältlich nach einem Verfahren gemäß Anspruch 8 oder 9.

11. Katalysatorformkörper gemäß einem der Ansprüche 5, 6, 7 oder 10, wobei das Cu/Zn-Atomverhältnis 15:85 bis 85:15, bevorzugt 60:40 bis 75:25 beträgt.

12. Katalysatorformkörper gemäß einem der Ansprüche 5, 6, 7, 10 oder 11, wobei das Zn/Al-Atomverhältnis 60:40 bis 80:20, bevorzugt 70:30 bis 80:20 beträgt.

13. Verwendung eines Katalysatorformkörpers gemäß einem der Ansprüche 5, 6, 7, 10, 11 oder 12 zur Synthese von Methanol aus Synthesegas enthaltend CO₂, CO, und H₂, vorzugsweise bei einer Raumgeschwindigkeit im Bereich von 2.000 bis 22.000 h⁻¹, einem Druck im Bereich von 60 bis 100 bar, und/oder bei einer Temperatur im Bereich von 200°C bis 300°C.

14. Verwendung eines Katalysatorformkörpers gemäß einem der Ansprüche 5, 6, 7, 10, 11 oder 12 zur Konvertierung von CO zu CO₂, vorzugsweise zur Tieftemperatur-Konvertierung von CO zu CO₂.

## Claims

1. Process for producing a shaped catalyst body containing copper, zinc and aluminium, which comprises the following steps:
(a) combining of an alkaline solution, in particular a carbonate-containing precipitant,
with a copper-containing solution, which is obtainable by dissolving and/or suspending a copper compound, a zinc compound and an aluminium compound,
to give a precipitate;
(b) isolation, optionally washing and/or optionally drying of the precipitate to give a solid catalyst precursor;
(c) thermal treatment at a temperature in the range from 200°C to 600°C, preferably in the range from 270°C to 550°C, and particularly preferably in the range from 450°C to 500°C, of the solid catalyst precursor obtained in step (b) to give a mixed oxide having a BET surface area according to DIN 66132 in the range from 80 m²/g to 140 m²/g, more preferably in the range from 85 m²/g to 120 m²/g, particularly preferably in the range from 90 m²/g to 110 m²/g;
(d) mixing of solid catalyst precursor obtained in step (b) with mixed oxide obtained in step (c) in a weight ratio of solid catalyst precursor to mixed oxide in the range from 2:98 to 20:80, preferably in the range from 5:95 to 15:85, more preferably in the range from 10:90 to 15:85, to give a mixture; and
(e) tableting of the mixture obtained in step (d), wherein the shaped catalyst body has a lateral compressive strength based on the pellet weight measured according to DIN EN 1094-5 of 500 N/g or more, preferably 600 N/g or more, in particular in the range from 600 to 900 N/g.

2. Process according to Claim 1, wherein the copper-containing shaped catalyst body has a loss on ignition determined according to the method described on pages 16 and 17 of the description of 7.5% by weight or less, preferably 5.5% by weight or less, in particular in the range from 0.1 to 4.0% by weight.

3. Process according to Claim 1 or 2, wherein the copper-containing solution in step (a) comprises an aluminium hydroxide sol.

4. Process according to any of Claims 1 to 3, wherein the copper-containing solution has a pH of ≤ 3.0.

5. Shaped catalyst body obtainable by a process according to any of Claims 1 to 4.

6. Shaped catalyst body according to Claim 5 which has a lateral compressive strength based on the pellet weight measured according to DIN EN 1094-5 in the range from 600 N/g to 1300 N/g, in particular in the range from 600 to 900 N/g.

7. Shaped catalyst body according to Claim 5 or 6 which has a volume shrinkage due to reduction, preferably due to reduction in a stream of hydrogen, of 6% or less, preferably 5% or less.

8. Process according to any of Claims 1 to 4, which comprises the following further step:
(f) reduction of the tableted mixture obtained in step (e).

9. Process according to Claim 8, wherein reduction is carried out by means of hydrogen.

10. Shaped catalyst body obtainable by a process according to Claim 8 or 9.

11. Shaped catalyst body according to any of Claims 5, 6, 7, or 10, wherein the Cu/Zn atomic ratio is from 15:85 to 85:15, preferably from 60:40 to 75:25.

12. Shaped catalyst body according to any of Claims 5, 6, 7, 10 or 11, wherein the Zn/Al atomic ratio is from 60:40 to 80:20, preferably from 70:30 to 80:20.

13. Use of a shaped catalyst body according to any of Claims 5, 6, 7, 10, 11 or 12 for the synthesis of methanol from synthesis gas containing CO₂, CO and H₂, preferably at a space velocity in the range from 2000 to 22 000 h⁻¹, a pressure in the range from 60 to 100 bar and/or a temperature in the range from 200°C to 300°C.

14. Use of a shaped catalyst body according to any of Claims 5, 6, 7, 10, 11 or 12 for the conversion of CO into CO₂, preferably for the low-temperature conversion of CO into

## Revendications

1. Procédé pour la préparation d'un corps moulé catalytique, contenant du cuivre, du zinc et de l'aluminium, comprenant les étapes suivantes :
(a) rassemblement d'une solution alcaline, en particulier d'un agent de précipitation contenant du carbonate, avec une solution contenant du cuivre, qui peut être obtenue par dissolution et/ou mise en suspension d'un composé du cuivre, d'un composé du zinc et d'un composé de l'aluminium, pour obtenir un précipité ;
(b) séparation, le cas échéant lavage et/ou le cas échéant séchage du précipité pour obtenir un précurseur catalytique solide ;
(c) traitement thermique du précurseur catalytique solide obtenu dans l'étape (b) à une température dans la plage de 200°C à 600°C, de préférence dans la plage de 270°C à 550°C et de manière particulièrement préférée dans la plage de 450°C à 500°C, pour obtenir un oxyde mixte présentant une surface BET selon la norme DIN 66132 dans la plage de 80 m²/g à 140 m²/g, de préférence dans la plage de 85 m²/g à 120 m²/g, de manière particulièrement préférée dans la plage de 90 m²/g à 110 m²/g ;
(d) mélange du précurseur catalytique solide obtenu dans l'étape (b) avec l'oxyde mixte obtenu dans l'étape (c) dans un rapport pondéral de précurseur catalytique solide à oxyde mixte dans la plage de 2:98 à 20:80, de préférence dans la plage de 5:95 à 15:85, plus préférablement dans la plage de 10:90 à 15:85 pour obtenir un mélange ; et
(e) compression du mélange obtenu dans l'étape (d), le corps moulé catalytique présentant une résistance à la compression latérale, rapportée au poids du comprimé, mesurée selon la norme DIN EN 1094-5, de 500 N/g ou plus, de préférence de 600 N/g ou plus, en particulier dans la plage de 600 à 900 N/g.

2. Procédé selon la revendication 1, le corps moulé catalytique contenant du cuivre présentant une perte à la calcination, déterminée selon la méthode décrite aux pages 16 et 17 de la description, de 7,5% en poids ou moins, de préférence de 5,5% en poids ou moins, en particulier dans la plage de 0,1 à 4,0% en poids.

3. Procédé selon la revendication 1 ou 2, la solution contenant du cuivre dans l'étape (a) comprenant un sol d'hydroxyde d'aluminium.

4. Procédé selon l'une quelconque des revendications 1 à 3, la solution contenant du cuivre présentant une valeur de pH ≤ 3,0.

5. Corps moulé catalytique, pouvant être obtenu selon un procédé selon l'une quelconque des revendications 1 à 4.

6. Corps moulé catalytique selon la revendication 5, qui présente une résistance à la compression latérale, rapportée au poids du comprimé, mesurée selon la norme DIN EN 1094-5, de 600 N/g à 1300 N/g, en particulier dans la plage de 600 N/g à 900 N/g.

7. Corps moulé catalytique selon la revendication 5 ou 6, qui présente un retrait volumique par réduction, de préférence par réduction dans un flux hydrogène, de 6% ou moins, de préférence de 5% ou moins.

8. Procédé selon l'une quelconque des revendications 1 à 4, comprenant l'étape supplémentaire suivante :
(f) réduction du mélange comprimé obtenu dans l'étape (e).

9. Procédé selon la revendication 8, la réduction ayant lieu avec de l'hydrogène.

10. Corps moulé catalytique, pouvant être obtenu selon un procédé selon la revendication 8 ou 9.

11. Corps moulé catalytique selon l'une quelconque des revendications 5, 6, 7 ou 10, le rapport atomique Cu/Zn valant 15:85 à 85:15, de préférence 60:40 à 75:25.

12. Corps moulé catalytique selon l'une quelconque des revendications 5, 6, 7, 10 ou 11, le rapport atomique Zn/Al valant 60:40 à 80:20, de préférence 70:30 à 80:20.

13. Utilisation d'un corps moulé catalytique selon l'une quelconque des revendications 5, 6, 7, 10, 11 ou 12 pour la synthèse de méthanol à partir d'un gaz de synthèse contenant CO₂, CO et H₂, de préférence à une vitesse spatiale dans la plage de 2000 à 22.000 h⁻¹, à une pression dans la plage de 60 à 100 bars et/ou à une température dans la plage de 200°C à 300°C.

14. Utilisation d'un corps moulé catalytique selon l'une quelconque des revendications 5, 6, 7, 10, 11 ou 12 pour la conversion de CO en CO₂, de préférence pour la conversion à basse température de CO en
